# EUROPEAN PATENT APPLICATION

(11) **EP 2 599 454 A1**
(43) Date of publication of application: **05.06.2013**
(21) Application number: 11009420.8
(22) Date of filing: 29.11.2011
(51) Int. Cl.: A61B 17/16, A61B 17/70

(54) **Spine fixation installation system**

(71) Applicant: spontech spine intelligence group AG, 70182 Stuttgart (DE)
(72) Inventor: Copf, Franz, 70184 Stuttgart (DE)
(74) Representative: Schwanhäußer, Gernot

(57) **Abstract**

A spine fixation system for fixing two vertebrae comprises a rod (12) and a plurality of fasteners (14) that are configured to be secured to the vertebrae (V1, V2, V3). The system further comprises a plurality of connectors (18) that are attached to the fasteners (14), a seat member (20) configured to be connected to the rod (12), and a first flat abutment face. The system further comprises a drill that is configured to produce a bore in the vertebrae and a material abrading tool which is configured to produce a second flat abutment face on the vertebrae such that the second abutment face at least substantially surrounds the bore and abuts seamlessly on the first abutment face if the fastener and the connector are attached to the respective vertebra.

## Description

### BACKGROUND OF THE INVENTION

### 1. Field of the Invention

The present invention generally relates to spine fixation systems that are used for the surgical treatment of spinal disorders which may require correction, stabilization, adjustment or fixation of the spinal column. More particularly, the invention relates to a spine fixation installation system comprising a computer, a spine fixation system and a rod bending device.

### 2. Description of Related Art

Various types of spinal column disorders are known and include scoliosis (abnormal curvature or rotation of vertebrae relative to the plane of the spine), kyphosis (abnormal backward curvature of the spine) and spondylolisthesis (forward displacement of a lumber vertebra), all of which involve a "misalignment" of the spinal column. Patients who suffer from such conditions usually experience extreme debilitating pain and physical deformity due to the condition. In severe cases treatments for these conditions have used a technique known as fusion with spinal fixation which results in the mechanical immobilization of areas of the spine and the eventual fusion of the vertebrae in the regions treated. In less severe cases treatment comprises decompression of the affected nerves and fusion of the vertebrae involved.

Fusion, however, is not usually successful unless the vertebrae are also fixed for a time period by a mechanical device installed internally during surgery. This allows the fused bone time to heal. Numerous mechanical systems have been proposed for this purpose. Screw and rod systems and screw and plate systems are commonly used to this purpose. The former system typically uses a rigid rod secured to the spine by screws inserted in the pedicles for holding the rod. The rod is usually bent to the desired configuration, and this both manipulates and holds the vertebrae in that same configuration until the fusion process can permanently accomplish the same thing.

If the screws in such screw and rod systems are not reliably secured in the vertebrae, the latter may fuse together in an undesired mutual relationship. This may give rise to continued pain and discomfort for the patient. The reason why screws tend to get loose is that the pedicles of the vertebrae (or other portions of the vertebrae to which the screws are attached) have only a relatively thin layer of compact bone material which is able to give the screw a sufficient stability. The inside portions of the bone has a much lower density so that the screws are prone to tilting movements around axes running perpendicular to the longitudinal direction of the screw and intersecting the outer layer of compact bone material.

### SUMMARY OF THE INVENTION

It is therefore an object of the present invention to improve the spine fixation installation such that the screws, bone anchors or similar fasteners can be more reliably secured to the vertebrae.

In accordance with the present invention this object is achieved by a spine fixation system for fixing two vertebrae. The system comprises a rod and a plurality of fasteners that are configured to be secured to the vertebrae, wherein each fastener comprises an elongated member having a distal end and a proximal end. The system further comprises a plurality of connectors. Each connector is attached to, or is capable of being attached to, the proximal end of one of the fasteners and comprises a seat member that is configured to be connected to the rod. The connector has a first flat abutment face pointing towards the distal end of the fastener. The system comprises also a drill that is configured to produce a bore in the vertebrae, wherein each bore is sized to receive one of the fasteners. A material abrading tool is provided which is configured to produce a second flat abutment face on the vertebrae such that the second abutment face at least substantially surrounds the bore and abuts seamlessly on the first abutment face if the fastener and the connector are attached to the respective vertebra.

The invention is based on the perception that only with an enlarged contact area between the compact bone tissue on the one hand and the fasteners and connectors on the other hand it is possible to achieve a reliable attachment of the fasteners and connectors to the vertebrae. If the first abutment face and the second abutment face cooperate with each other by abutting seamlessly on each other, this contact area is so large that the fastener cannot perform tilting movements, in particular if this contact area is arranged perpendicular to a longitudinal direction of the elongated member of the fasteners.

The at least one fastener may be a pedicle screw, but other fasteners, for example anchors which are configured to be cemented into bores, are envisaged as well.

In one embodiment the system comprises a guide element that is configured to guide the tool relatively to the bore. This guide element has to be attached to the vertebra in a way so that a defined positional relationship with respect to the bore is obtained.

In the simplest case the guide element is a rod which is configured to be inserted into the bore. The material abrading tool then comprises a sleeve having an inner diameter that corresponds to an outer diameter of the rod. The tool further comprises a tool head having a flat abrading face. This provides a very simple, but efficient guiding of the material abrading tool relatively to the bore.

If the material abrading tool comprises a sleeve and a tool head, the abrading face may have an annular shape having an axis of symmetry which coincides with a longitudinal axis of the sleeve.

In some embodiments the guide element comprises a stop that delimits an axial range of movement of the tool with respect to the guide element. This helps to prevent that the tool removes too much compact bone material so that the less dense spongy bone is exposed and forms the second flat abutment face.

If the guide element is formed by a rod, the stop may comprise a projection, for example an annular collar, that projects radially from the rod.

In that case the tool head of the material abrading tool may comprise a recess that is sized so that the tool head can slide over the projection.

In one embodiment the connector comprises at least one spacer element on which the first flat abutment face is formed and which is configured to be inserted between a main body of the connector and the fastener so as to enlarge the distance between the respective vertebra and a top end of the connector if the fastener and the connector are attached to the vertebra.

Such additional spacer elements are useful because the invention requires an immediate contact between the connector and the vertebrae. Therefore it is not an option to secure the fastener at an arbitrary axial position in a bore so that the seat member is positioned at a desired height above the vertebra. However, with a spacer element a height adjustment of the seat member is possible. This particularly applies if the system comprises not only one, but a plurality of spacer elements having different thicknesses in a direction along a longitudinal axis of a bore provided in the spacer element.

As outlined above, it is preferable if the contact area between the connector and the vertebra is as large as possible. Therefore the total area of the first flat abutment face may be at least two times, preferably at least five times, as large as a maximum cross sectional area of the elongated member of the fastener.

Subject of the present invention is also a material abrading tool for installing a spine fixation system. The tool comprises a sleeve and a tool head having a flat material abrading face. The tool is sized an configured to be inserted through an access channel, which has been prepared in a patient to be treated, such that the sleeve guides over a guide rod that is inserted in a bore provided in a vertebra of the patient.

The material abrading face may have an annular shape having an axis of symmetry which coincides with a longitudinal axis of a sleeve.

### BRIEF DESCRIPTION OF THE DRAWINGS

Various features and advantages of the present invention may be more readily understood with reference to the following detailed description taken in conjunction with the accompanying drawing in which:
- FIG. 1: is a partial perspective view of a prior art spine fixation system;
- FIG. 2: is a side view of a pedicle screw and a connector after installation according to the prior art;
- FIG. 3: is a sectional view of a portion of a pedicle illustrating the insertion of a guide rod into a bore according to the present invention;
- FIG. 4: is a sectional view similar to FIG. 3, but with the guide rod fully inserted into the bore;
- FIG. 5: is a perspective view of a material abrading tool in accordance with the present invention;
- FIG. 6: is a partial view of the tool shown in FIG. 5 applied to the pedicle as shown in FIG. 4;
- Fig. 7: shows the portion of the pedicle after the removal of bone tissue by the tool shown in FIG. 5;
- FIG. 8: is a side view of a spine fixation system in accordance with the present invention in a representation similar to FIG. 2;
- FIG. 9: is a bottom view of a main body of a connector which is part of the spine fixation system shown in FIG. 8;
- FIG. 10: shows a guide rod according to a second embodiment having a stop element for delimiting the axial movement of the material abrading tool in a representation similar to FIG. 4;
- FIG. 11: illustrates the abrading process using a modified material abrading tool that cooperates with the stop element as shown in FIG. 10;
- FIG. 12: corresponds to FIG. 11, but with the abrading process being further continued;
- FIG. 13: illustrates the result of the abrading process;
- FIG. 14: illustrates, in a representation similar to FIG. 8, the spine fixation system after installation according to the second embodiment;
- FIG. 15: shows, in a representation similar to FIG. 14, a spine fixation system according to a third embodiment, in which the connector comprises an additional spacer element.

### DESCRIPTION OF PREFERRED EMBODIMENTS

### 1. Prior Art Spine Fixation System

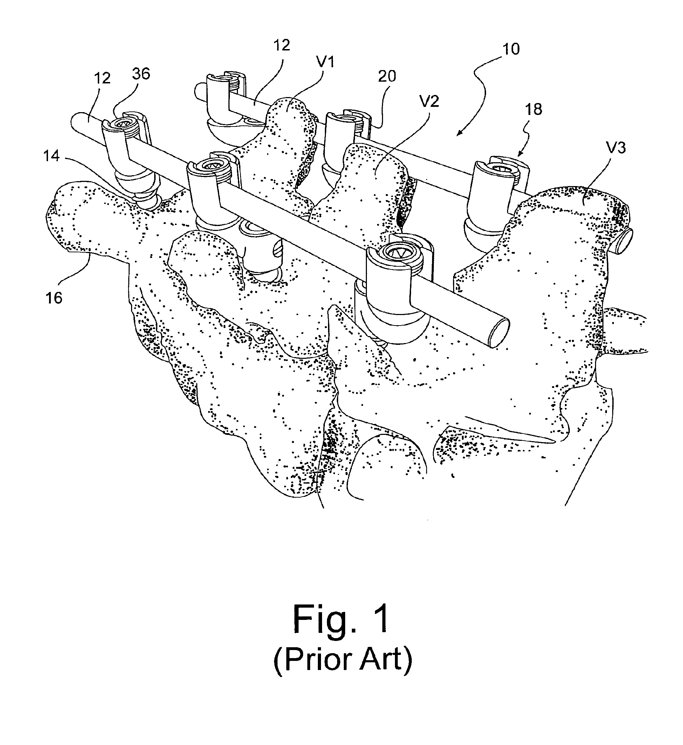

FIG. 1 is a perspective view of a portion of a prior art spine fixation system 10 as it is known from WO 2010/108665 A2 and after having been installed in a segment of a human spine. This segment comprises three vertebrae V1, V2, V3 which are arranged one behind the other along the vertebral column. For the sake of simplicity the vertebrae adjacent to this segment are not shown in FIG. 1.

The spine fixation system 10 comprises two rods 12 which extend on either side of the spine segment along a longitudinal direction of the spinal column. The rods 12 are rigid and curved elements made of a suitable metal, for example titanium, and have a circular cross section. However, it is also known to use rods having other than circular cross-sections, for example an oval or polygonal cross-section. In the embodiment shown the rods 12 are only slightly curved so that the curvature cannot be readily seen in the perspective view of FIG. 1. Depending on the position of the spine segment along the human spine, the curvature may be stronger.

The rods 12 are fastened to the vertebrae V1, V2, V3 with the help of pedicle screws 14, which are secured in pedicles 16 of the vertebrae V1, V2 V3, and connectors 18 that connect the pedicle screws 14 to the rods 12. Each connector 18 has a seat member formed by a tulip 20 that has various degrees of freedom so that it can be positioned and thereafter be fixed in many different ways. This makes it possible to connect the curved rods 12 to the pedicle screws 14 also in those cases in which the curved rods 12 do not exactly extend along the curved line that is defined by the positions of the pedicle screws 14.

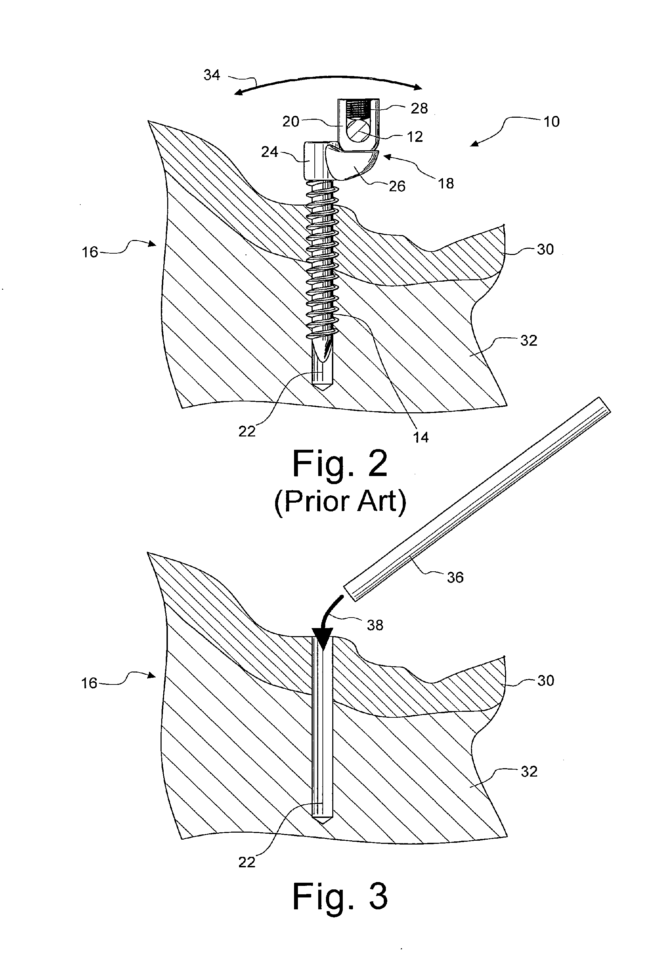

FIG. 2 is a side view of one of the pedicle screws 14 secured in one of the pedicles 16. The pedicle screw 14 is attached to one of the connectors 18 and is screwed in a bore 22 that has been drilled beforehand. The connector 18 comprises a main body 24 having an at least substantially cylindrical shape and a tulip base 26 which supports the tulip 20. The tulip base 26 projects radially from the cylindrical main body 24 and has an hemispherical recess in which the tulip 20 is received. A fixing screw 28 is screwed into the tulip 20 so as to fix the rod 12 in the tulip 20. The fixing screw 28 also fixes the tulip 20 with respect to the tulip base 26. More details with respect to the connector 18 are described in the aforementioned WO 2010/108665 A2.

The pedicle 16 is a short thick process which projects dorsally, one on either side, from the superior part of the vertebral body and connects this body to the arch. As other bones, the pedicle has a hard outer layer 30 which is composed of compact bone tissue. The outer layer 30 covers spongy bone 32 which is an open cell porous network having a much lower density than the compact bone tissue of the hard outer layer 30. Therefore the pedicle screw 14 is mainly held on the spot by the compact bone tissue of the hard outer layer 30, whereas the softer spongy bone 32 contributes to the stability of the connection between the pedicle screw 14 and the vertebra V only to a much smaller extent.

Particularly if the hard outer layer 30 is thin, the pedicle screw 14 is therefore prone to tilt around axes running perpendicular to the longitudinal direction of the pedicle screw 14, as this is indicated by a double arrow 34 in FIG. 2. Such tilt movements severely compromise the stabilizing effect of the entire spine fixation system 10 and may eventually result in vertebrae V1, V2, V3 that do not fuse in the relative arrangement that was originally intended.

### 2. First Embodiment of Spine Fixation System

Referring now to FIGS. 3 to 8, a first embodiment of a spine fixation system in accordance with the present invention will be described in the following. These figures show, in a representation similar to FIG. 2, a portion of one of the pedicles 16 where the spine fixation system 10 shall be installed, at subsequent steps during the installation procedure.

FIG. 3 shows a portion of the pedicle 16 into which the bore 22 has been drilled with the help of a conventional bone drill. In a next step a guide rod 36 is inserted into the bore 22, as this is indicated by an arrow 38. The guide rod 36 has a cylindrical shape with an outer diameter which equals the diameter of the bore 22 so that it can be inserted with loose fit into the bore 22.

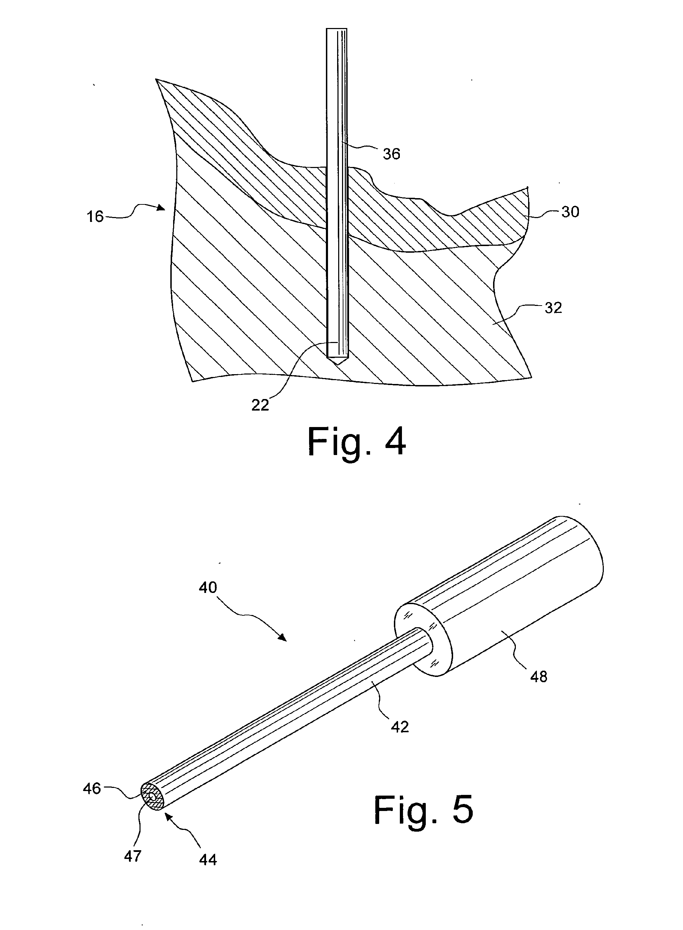

FIG. 4 shows the guide rod 36 after being completely inserted into the bore 22.

In a next step a flat abutment surface is formed on the pedicle 16 with the help of a material abrading tool 40 which is shown in FIG. 5 in a perspective view. The tool 40 comprises a sleeve 42 having a central channel 47 having a diameter that corresponds to the diameter of the guide rod 36 so that the sleeve 42 can be slid over the guide rod 36 with loose fit. At a distal end of the sleeve 42 the tool 40 has a tool head 44 comprising a flat abrading face 46. The abrading face is serrate or otherwise roughened so that it is able to abrade bone tissue. At the proximal end of the sleeve 42 a handle 48 is rigidly attached to the sleeve 42.

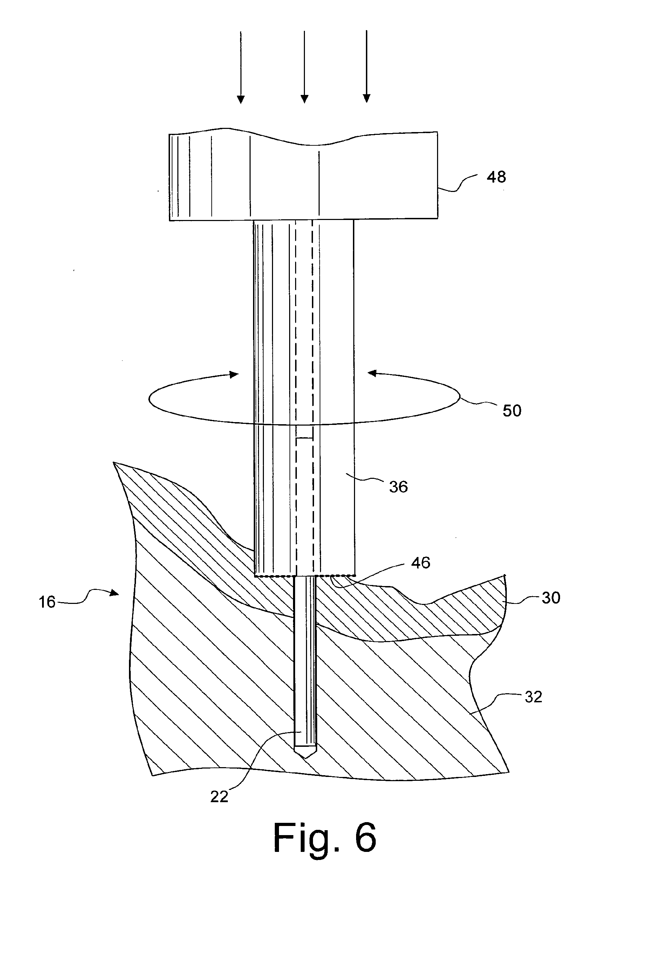

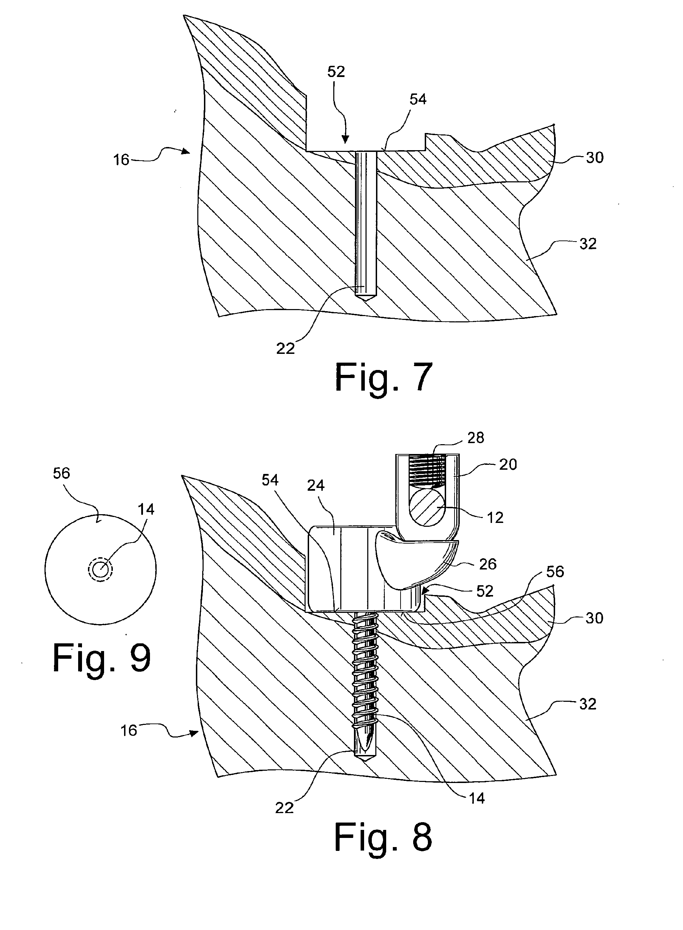

FIG. 6 illustrates the application of the material abrading tool 40. First the tool 40 is slid over the guide rod 36 so that the latter enters the channel 47 defined by the sleeve 42. Then the tool 40 is rotated, as indicated by double arrow 50 in FIG. 6, while simultaneously pushing it downward so that the abrading face 46 is pressed against the outer hard layer 30 of the pedicle 16. In this manner a certain amount of compact bone material is abraded, thereby producing a cylindrical recess 52 and a flat abutment face 54 surrounding the bore 22, as this can be seen in FIG. 7 in a final state after the abrading process has been terminated and the guide rod 36 has been removed.

The surgeon should continue with the abrading process only until the flat abutment face 54 has approximately the same area as the abrading face 46 at the tool head 44. If the abrading process is continued longer, there may be a risk that the hard outer layer 30 of the pedicle 16 is completely removed so that the flat abutment face 54 would be formed on the weak spongy bone 32. This would compromise the stability of a later attachment of the pedicle screw 14 and the connector 18 to the pedicle 16.

Therefore, if the surgeon notices that the abrading face 46 gets in contact with spongy bone 32, he should stop the abrading process. Then the flat abutment face 54 may have a total area which is smaller than the area of the abrading face 46, but this usually still suffices to achieve the desired stabilizing effect which will be described in the following with reference to FIG. 8.

FIG. 8 shows the pedicle screw 14 and a connector 18 after the installation has been completed. The connector is specifically adapted to the recess 52 in that the main body 24 of the connector 18 has a diameter that is only slightly smaller than the diameter of the abrading face 46. Thus the main body 24 fits into the recess 52 which has been produced with the help of the tool 40. Furthermore, the main body 24 of the connector 18 has a flat abutment face 56 on its underside that points towards the distal end of the pedicle screw 14 in the assembled configuration. As can be seen in the bottom view of the main body 24 shown in FIG. 9, the abutment face 56 has an area which is significantly larger than the maximum cross sectional area 58 of the pedicle screw 14. In the embodiment shown the ratio is about 20.

In the installed configuration shown in FIG. 8, the abutment face 56 of the connector 18 on the one hand and the hard abutment face 54 formed at the at the bottom of the recess 52 on the other hand seamlessly abut. Thus the connector 18 is very effectively secured against tilting, and a very rigid and reliable attachment of the pedicle screw 14 to the pedicle 16 is achieved.

### 3. Second Embodiment of Spine Fixation System

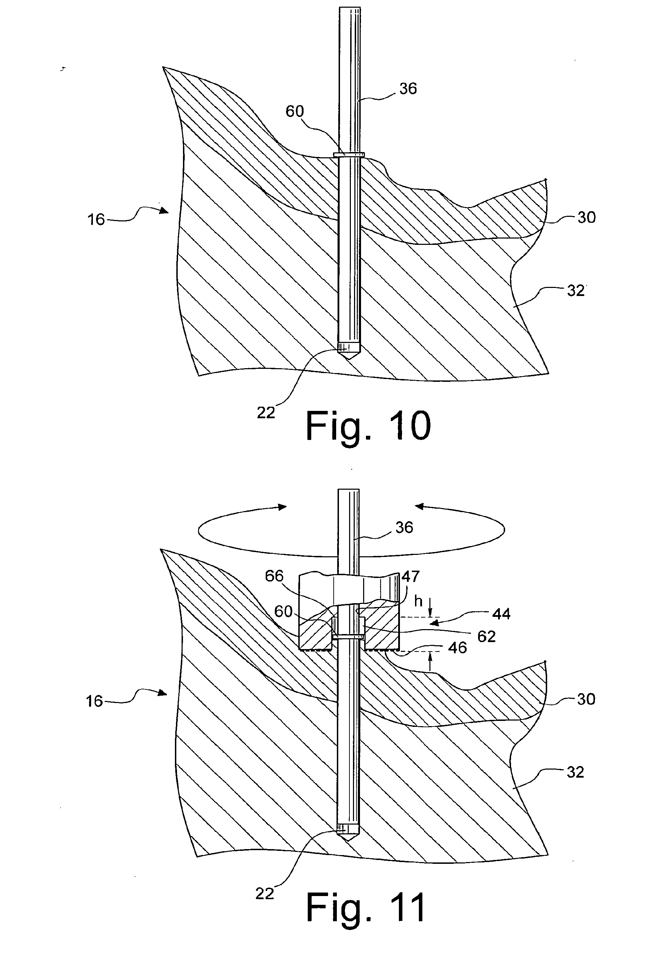

FIGS. 10 to 14 show, in illustrations similar to FIGS. 3 to 8, a second embodiment of the present invention in which a stop prevents the tool 40 from abrading too much compacted bone of the hard outer layer 30,.

Referring first to FIG. 10, the guide rod 36 comprises an annular collar 60 forming a stop that radially projects from the guide rod 36. The collar 60 rests on the pedicle 16 and delimits, in cooperation with a recess formed in the tool head 44, the axial range of movement of the tool 40 with respect to the guide rod 36.

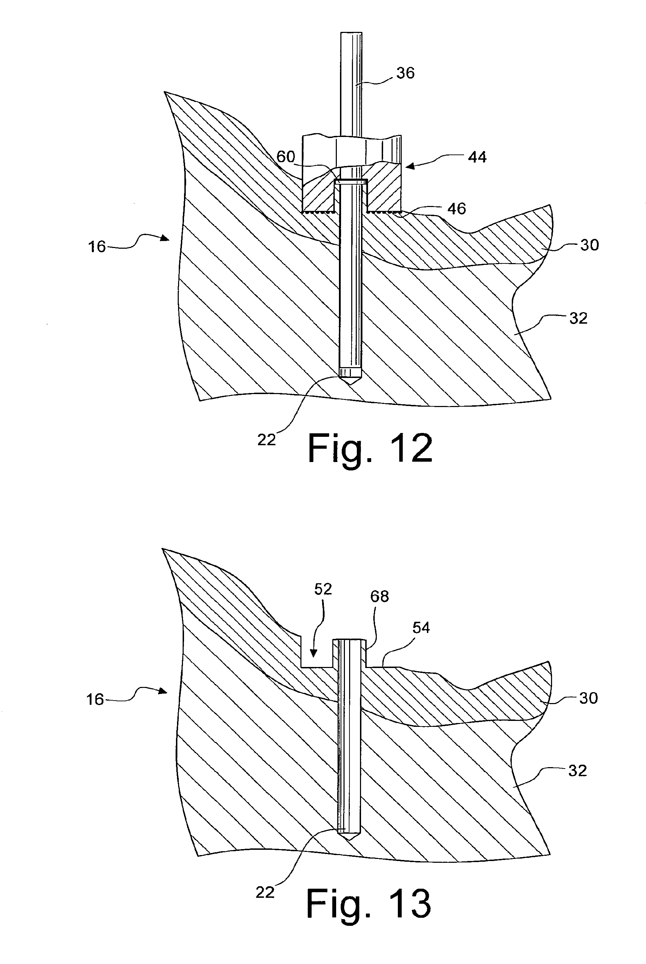

This can be seen in FIG. 11 which shows the tool head 44 while it is rotated so as to abrade bone tissue from the surroundings of the bore 22. In this embodiment the tool head 44 is provided with a cylindrical recess 62 which is aligned coaxially with the channel 47 of the sleeve 42 through which the guide rod 36 extends. The diameter of the recess 62 is sized so that the tool head 44 can slide over the collar 60 projecting from the guide rod 36 until the collar 60 abuts on an annular abutment face 66 formed at the bottom of the recess 62. Thus the maximum axial range of movement of the tool 40 relative to the collar 60 is determined by the height h of the recess 62. This is also illustrated in FIG. 12 which shows the tool head 44 at its end position when the annular abutment face 66 abuts on the collar 60 resting on the pedicle 16.

If tools 40 with different height h of the recess 62 are provided, or if the tool head 44 can be detached and replaced by a tool head with a differently sized recess 62, it is possible to vary the maximum depth of the recess 52 that is produced in the pedicle 16 with the help of the tool 40.

After removal of the tool 40 and the guide rod 36, a sleeve-like bone portion 68 remains that projects upward from the flat abutment face 54. This portion 68 may be removed using a knife, a hammer or another material abrading tool.

Then the same connector 18 may be used as the one that has been explained above with reference to FIGS. 8 and 9.

### 4. Third Embodiment of Spine Fixation System

In the prior art solutions the position of the tulip 20 above the pedicle 16 can be easily adjusted by turning the pedicle screw 14 inside the bore 22, because the connector 18 does not rest directly on the pedicle 16, as this is shown in FIG. 2. With the present invention, however, the connector 18 rests with its flat abutment face 56 on the flat abutment face 54 that had been produced with the help of the tool 40. Thus the height of the tulip 20 above the pedicle 16 cannot be adjusted, without forfeiting the desired stabilizing effect, by simply turning the pedicle screw 14.

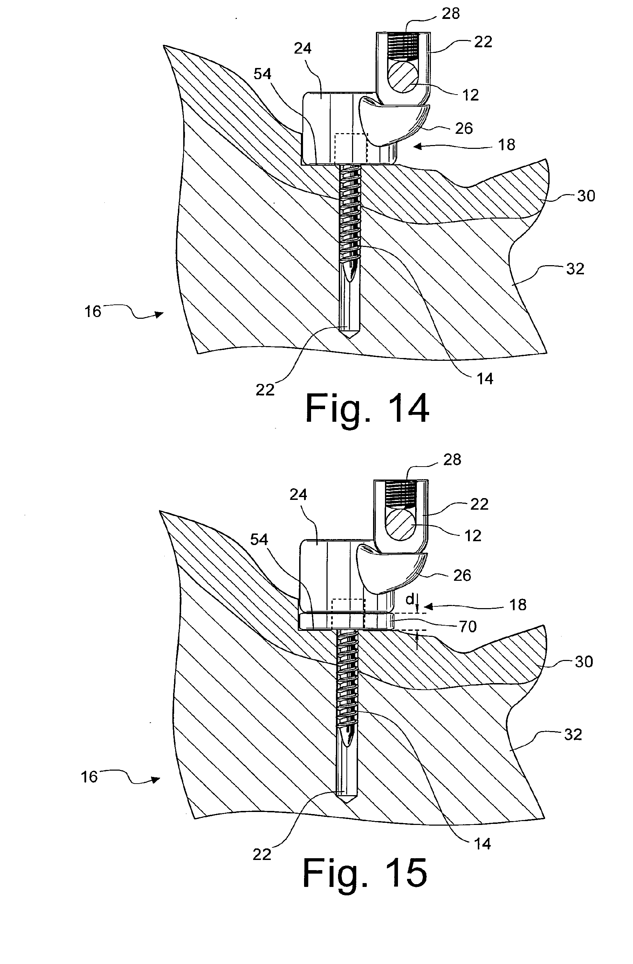

The present invention therefore envisages using one or more spacer elements 70 that are sandwiched between the main body 24 of the connector 18 and the flat abutment face 54, as this is shown in FIG. 15. The spacer element 70 comprises a bore, through which the pedicle screw 14 extends, and has generally the shape of a circular disc. One side of the spacer element forms the abutment face 56 of the connector 18, and the opposite face abuts on the main body 24 of the connector. The thickness d of the spacer element 70 determines the distance between the tulip 20 and the pedicle 16. This distance may be adjusted by using one or more spacer elements 70 having different thicknesses d. The spacer element 70 may therefore be considered as forming a detachable part of the connector 18.

The above description of the preferred embodiments has been given by way of example. From the disclosure given, those skilled in the art will not only understand the present invention and its attendant advantages, but will also find apparent various changes and modifications to the structures and methods disclosed. The applicant seeks, therefore, to cover all such changes and modifications as fall within the spirit and scope of the invention, as defined by the appended claims, and equivalents thereof.

## Claims

1. A spine fixation system for fixing two vertebrae, comprising
a) a rod (12),
b) a plurality of fasteners (14) that are configured to be secured to the vertebrae (V1, V2, V3), wherein each fastener comprises an elongated member having a distal end and a proximal end,
c) a plurality of connectors (18), wherein each connector
- is attached to, or is capable of being attached to, the proximal end of one of the fasteners (14) and
- comprises a seat member (20) that is configured to be connected to the rod (12), and
- has a first flat abutment face pointing towards the distal end of the fastener,
d) a drill that is configured to produce a bore in the vertebrae, wherein each bore is sized to receive one of the fasteners,
e) a material abrading tool which is configured to produce a second flat abutment face on the vertebrae such that the second abutment face at least substantially surrounds the bore and abuts seamlessly on the first abutment face if the fastener and the connector are attached to the respective vertebra.

2. The system of claim 1, wherein at least one fastener is a pedicle screw.

3. The system of claim 1, wherein at least one fastener is an anchor which is configured to be cemented into the bore.

4. The system of any of the preceding claims, comprising a guide element that is configured to guide the tool relatively to the bore.

5. The system of claim 4, wherein the guide element is a rod which is configured to be inserted into the bore, and wherein the material abrading tool comprises a sleeve, which has an inner diameter that corresponds to an outer diameter of the rod, and a tool head having a flat abrading face.

6. The system of claim 5, wherein the abrading face has an annular shape having an axis of symmetry which coincides with a longitudinal axis of the sleeve.

7. The system of any of claims 4 to 6, wherein the guide element comprises a stop that delimits an axial range of movement of the tool with respect to the guide element.

8. The system of claim 7 and comprising the features of claim 5 or 6, wherein the stop comprises a projection that projects radially from the rod.

9. The system of claim 8, wherein the projection is an annular collar.

10. The system of claim 8 or 9, wherein at least the tool head comprises a recess that is sized so that the tool head can slide over the projection.

11. The system of any of the preceding claims, wherein the connector comprises at least one spacer element on which the first flat abutment face is formed and which is configured to be inserted between a main body of the connector and the fastener so as to enlarge the distance between the respective vertebra and a top end of the connector if the fastener and the connector are attached to the vertebra.

12. The system of claims 11, wherein the at least one spacer element comprises a bore.

13. The system of claim 12, comprising a plurality of spacer elements having different thicknesses in a direction along a longitudinal axis of the bore.

14. The system of any of the preceding claims, wherein the total area of the first flat abutment face is at least two times, preferably at least 5 times, as large, as a maximum cross sectional area of the elongated member of the fastener.

15. A spine fixation system for fixing two vertebrae, comprising
a) a rod (12),
b) a plurality of fasteners (14) that are configured to be secured to the vertebrae (V1, V2, V3),
c) a plurality of connectors (18), wherein each connector
- is attached to, or is capable of being attached to, one of the fasteners (14) and
- is configured to be connected to the rod (12), and
- has a first flat abutment face pointing towards the vertebra after installation.

16. The system comprising the features of any of claims 2 to 14.

17. A material abrading tool for installing a spine fixation system, said tool comprising a sleeve and a tool head having a flat material abrading face, wherein the tool is sized and configured to be inserted through an access channel, which has been prepared in a patient to be treated, such that the sleeve glides over a guide rod that is inserted in a bore provided in a vertebra of the patient.

18. The tool of claim 16, wherein the material abrading face has an annular shape having an axis of symmetry which coincides with a longitudinal axis of the sleeve.

19. A method of installing a spine fixation system for fixing two vertebrae, comprising the following steps:
a) providing a plurality of fasteners (14) that are configured to be secured to the vertebrae (V1, V2, V3),
b) providing a plurality of connectors (18),
wherein each connector
- is attached to, or is capable of being attached to, the proximal end of one of the fasteners (14) and
- comprises a seat member (20) that is configured to be connected to the rod (12), and
- has a first flat abutment face;
c) drilling a bore in one of the vertebrae;
d) producing a second flat abutment face (12) on the one vertebra such that it at least substantially surrounds the bore;
e) installing one of the fasteners and one of the connectors by inserting the fastener into the bore until the first abutment face on the connector abuts seamlessly on the second abutment face on the vertebra.
